# EUROPEAN PATENT SPECIFICATION

(11) **EP 1 734 923 B1**
(45) Date of publication and mention of the grant of the patent: **21.03.2012**
(21) Application number: 05732636.5
(22) Date of filing: 24.03.2005
(51) Int. Cl.: A61K 9/00, A61K 47/02

(54) **ZINC PRESERVATIVE COMPOSITION AND METHOD OF USE**
ZINK-KONSERVIERUNGSZUSAMMENSETZUNG UND ANWENDUNGSVERFAHREN
COMPOSITION DE CONSERVATEUR A BASE DE ZINC ET PROCEDE D'UTILISATION

(30) Priority: 29.03.2004 US 812543
(43) Date of publication of application: 27.12.2006
(60) Divisional application: 10176715.0
(73) Proprietor: BAUSCH & LOMB INCORPORATED, Rochester, New York 14604-2701 (US)
(72) Inventor: XIA, Erning, Penfield, NY 14526 (US); SALAMONE, Joseph, C., Fairport, NY 14450 (US); BORAZJANI, Roya, N., Fairport, NY 14450 (US)
(74) Representative: Maiwald Patentanwalts GmbH
(86) International application number: PCT/US2005/009742
(87) International publication number: WO 2005/097067

(56) References cited:
- EP-A- 0 074 819
- EP-A- 0 420 798
- EP-A- 0 470 667
- EP-A- 0 546 728
- EP-A- 0 663 208
- WO-A-02/49615
- US-A- 5 607 678
- GRAHN BRUCE H ET AL: "Zinc and the eye" JOURNAL OF THE AMERICAN COLLEGE OF NUTRITION, vol. 20, no. 2 with Supplement, April 2001 (2001-04), pages 106-118, XP002334806 ISSN: 0731-5724 cited in the application

## Description

### FIELD OF THE INVENTION

The present invention relates to the methods and/or compositions, particularly ophthalmic compositions such as eye drop and contact lens treating solutions.

### BACKGROUND

The contact of eye tissue with bacteria may lead to various eye infections, such as microbial keratitis. The contact of eye tissue with bacteria may result when an ophthalmic solution contaminated with bacteria is instilled directly in the eye. Examples of such ophthalmic solutions instilled directly in the eye are eye drop solutions (for example, for treating dry eye) or contact lens drop solutions (for example, for conditioning a contact lens while worn). Additionally, eye tissue may be contacted with bacteria by placing a contact lens on the eye where the contact lens is contaminated with bacteria. The risk of eye infection is increased when bacteria is adhered to a contact lens, since the bacteria may remain in contact with eye tissue for a prolonged period of time.

For this reason, ophthalmic compositions, such as eye drop and contact lens treating solutions, conventionally include a preservative agent that acts to inhibit growth of bacteria and/or fungi, as well as other infectious organisms, in case the solution becomes contaminated with such organisms. For contact lens treating solutions, the preservative agents used to preserve the solution may also serve to disinfect contact lenses when rinsed or soaked with the solution. Alternately, ophthalmic compositions may include no preservative, but in such cases, the compositions are packaged in a special container that prevents contamination of the container contents, an example being single unit-dose packages where each dosage of solution is separately packaged.

Various preservative agents are known for use in ophthalmic compositions. Such preservative agents should have a broad spectrum of preservative activity and be non-irritating to the eye. However, many preservative agents have a tendency to irritate eye tissue, especially at higher concentrations. Therefore, it is generally advantageous to employ as low as possible concentration of preservative agent to avoid the risk of eye irritation.

U.S. Patent Nos. 6,323,165 and 6,274,133 disclose compositions and methods for blocking protein and/or lipid deposits on hydrophilic contact lenses with polyquaternium polymers or cationic cellulose polymers that bind to lenses and block the deposits from binding.

U.S. Patent No. 4,443,429 discloses the use in a contact lens disinfecting solution of a dimethyldiallylammonium chloride homopolymer commercially known as Merquat^{™} 100 (i.e., which has a molecular weight of about 10,000 to about 1,000,000). Preferred disinfecting solution concentrations were recited therein as 0.0004 wt.% to about 0.02 wt.% (4 ppm to 200 ppm).

WO 02/34308 discloses inhibiting adhesion of bacteria to the surface of a biomedical device, such as a contact lens, by binding a cationic polysaccharide to the surface of the device.

Pending U.S. Ser. Nos. 10/427,056 filed April 30, 2003, and 10/427,084 filed April 30, 2003, disclose the use of polycations such as Polymer JR (Polyquaternium-10) as a preservative-enhancing additive. Polymer JR was less of an irritant than traditional preservatives but enhanced the performance of the traditional preservative. U.S. Patent No. 5,460,834 discloses a physiological tear composition that may contain a zinc compound at a concentration having a minimum of about 0.005 and/or a maximum of about 0.015 mmol/L in addition to other salts known to be present in tears.

U.S. Patent No. 2,230,748 discloses an impregnating solution that prevents rot fungus in textiles or other organic materials. The impregnating solution includes sugar or sachcariferous substances. Soluble salts of zinc and copper are disclosed as ingredients in the solution.

A National Eye Institute Study entitled, NEI Study: Antioxidants and Zinc May Reduce AMD Risk, Review of Optometry, pp. 138-49, volume 6(1) (November 15, 2001), concluded that high levels of antioxidants and zinc was believed to significantly reduce the risk of advanced age related macular degeneration. Zinc was administered orally in the study.

Grahn, B.H. et al., Zinc and the Eye, Journal of the American College of Nutrition, vol. 20 (2) pp. 106-118 (April 2001) indicated that zinc played an important role in maintaining normal ocular function. Zinc was administered orally to patients. WO 02/49615 A2 relates to an artificial tear formulation comprising a combination of three demulcents in an amount of 0.5 to 5.0 w/v %, said combination including HPMC, a dextran, and glycerin; and water.

It would be desirable to provide an ophthalmic composition with enhanced preservative efficacy that is safe, convenient and economical to use and non-irritating to eye tissue. The present invention addresses these and other problems encountered in the art.

### SUMMARY OF THE INVENTION

The present invention relates to a composition that include 0.001 wt-% to 1 wt-% of a zinc compound and has
no primary preservative agent. The composition has the benefit of being adequately preserved without having a harsh physiological effect such as irritation or discomfort caused by at least some traditional preservative agents. The present invention relates to processes for manufacture, methods of enhancing compositions, and methods of use of the composition related to the above composition.

According to another embodiment, the composition further comprises a polycationic material including but not limited to a cationic cellulosic Polymer such as, for example, Polymer JR.

According to one embodiment, the composition is an ophthalmic solution that optionally includes at least one component selected from the group consisting of tonicity adjusting agents, buffering agents, chelating agents, pH adjusting agents, viscosity modifying agents, and therapeutic agents.

In one embodiment, the composition is an eye drop solution. In another embodiment, the composition is a contact lens treating solution. Typically, the composition is suitable for direct instillation in the eye without irritation to eye tissue.

### DETAILED DESCRIPTION OF THE INVENTION

The present invention relates to a composition that includes 0.001 wt-% to 1 wt-% of a zinc compound and has
10 primary preservative agent The composition has the benefit of being adequately preserved without having a harsh physiological effect such as irritation or discomfort caused by at least some traditional preservative agents. Likewise in another embodiment, there is an ophthalmic composition comprising 0.001 wt-% to 1 wt-% of a zinc compound. The ophthalmic composition comprises
no primary preservative agent.

The term "preservative" or like terms denotes agents included in the ophthalmic compositions for the purpose of inhibiting the growth of microorganisms in the product, thereby helping to maintain sterility of the composition. The term "preservative agent" denotes the specific active agent, which provides the preservative efficacy. As mentioned, the composition has no primary preservative agent. Primary preservative agents are defined as non-zinc containing compounds that derive their preservative activity through a chemical or physiochemical interaction with the microbial organisms.

The term "amount" as it pertains to a zinc compound is defined as an amount of a zinc compound that either dissolves completely in the composition or exceeds the saturation level by an amount that is not noticeable to the patient when the composition is applied to the patient's eye. Preferably, the zinc compound is completely dissolved.

According to this invention, there is one embodiment where the zinc compound is in the +2 valence state. In another embodiment, the composition has a minimum of about 0.001 wt.%, about 0.005 wt.%, about 0.01 wt.% or about 0.05 wt.% of a zinc compound per total weight of the composition and/or a maximum of about 1 wt.%, about 0.5 wt.%, about 0.1 wt.% or about 0.05 wt.% of the zinc compound per total weight of the composition. In one embodiment, the zinc compound is selected from the group comprising zinc citrate, zinc chloride, zinc acetate, zinc bromide, zinc fluoride, zinc iodide, zinc ammonium sulfate, zinc nitrate and zinc sulfate. Preferably, the zinc compound is selected from the group comprising zinc citrate and zinc chloride in one embodiment. Zinc citrate and zinc chloride can be obtained from Sigma-Aldrich Chemical Company, St. Louis, Missouri.

In one embodiment, the method further comprises adding a polycationic material. The compositions of the present invention include a polycationic material. The term "polycation" material denotes a material having multiple cationic moieties, such as quaternary ammonium groups, in the same molecule.

Many of the polycationic materials, by themselves, do not have sufficient preservative efficacy to adequately preserve an ophthalmic composition against a broad spectrum of microorganisms but can enhance preservative efficacy when used in conjunction with a soluble zinc compound. Illustrative polycationic materials include cationic polysaccharides, cationic proteins, cationic polynucleotides, cationic glycoproteins, cationic glycosaminoglycans and ionene polymers, having multiple cationic molecules in the same molecule.

In general, polyquaternium polymers suitable for use in the present invention are a well-known class of polymers of which many variations are commercially available. The polyquaternium polymer preferably includes an ophthalmologically suitable anionic organic or inorganic counterion. A preferred counterion may include, but are not limited to fluoride ions, chloride ions, bromide ions, iodide ions and the like.

For example, the polyquaterniums designated Polyquaternium-1 through Polyquatemium-44 (CTFA International Cosmetic Ingredient Dictionary) includes a number of materials that are useful, based on the present teachings, in the present invention. The polymerization techniques for the preparation of such materials are similarly well known to those skilled in the art and many variations of such techniques are similarly in practice in commerce.

In general, the polyquaternium polymers suitable for use in the present invention have a weight average molecular weight is a minimum of about 500 Daltons, about 1000 Daltons or about 2000 Daltons and/or a maximum of about 5,000,000 Daltons, about 500,000 Daltons or about 200,000 Daltons. One preferred class of polycationic materials is cationic polysaccharides, and especially, cationic cellulosic polymers. Specific examples of cationic cellulosic polymers include but are not limited to cellulosic polymers containing N,N-dimethylaminoethyl groups (either protonated or quatemized) and/or N,N-dimethylamino-2-hydroxylpropyl groups (either protonated or quaternized). Cationic cellulosic polymers are commercially available or can be prepared by methods known in the art. As an example, quaternary nitrogen-containing ethoxylated glucosides is prepared by reacting hydroxyethyl cellulose with a trimethylammonium-substituted epoxide, according to one embodiment.

In another embodiment, the composition has a minimum of about 0.001 wt.%, about 0.005 wt.%, about 0.01 wt.% and 0.05 wt.% and/or a maximum of about 0.5 wt.% about 0.1 wt.% or about 0.05 wt.% cationic cellulose polymer based upon the total weight of the composition.

Various preferred cationic cellulosic polymers are commercially available, for example water-soluble polymers available under the CTFA (Cosmetic, Toiletry, and Fragrance Association) designation Polyquatemium-10 (hereinafter defined as Polymer JR). Such polymers are commercially available under the tradename UCARE® Polymer from Amerchol Corp., Edison, N.J., USA. These polymers contain quatemized N,N-dimethylamino groups along the cellulosic polymer chain. Suitable cationic cellulosic materials have the following formula: 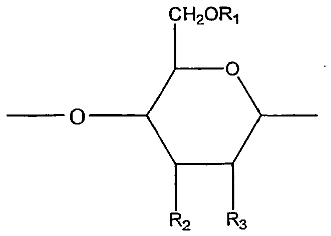 Wherein R₁ R₂ and R₃ are selected from H, derivatives of C₁-C₂₀ carboxylic acid, C₁-C₂₀ alkyl groups, C₁ to C₃ monohydric and dihydric alkanols, hydroxyethyl groups, hydroxypropyl groups, ethylene oxide groups, propylene oxide groups, phenyl groups, "Z" groups and combinations thereof. One or more of R₁ R₂ and R₃ is a Z group.

The nature of the "Z" groups is: 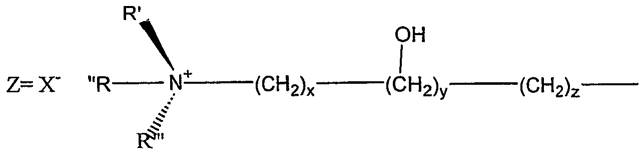 wherein:
R', R" and R"' can be H, CH₃, C₂H₅, CH₂CH₂OH and CH₂CH(OH)CH₂OH
x=0-5, y=0-4, and z=0-5
X = Cl⁻, Br⁻, I⁻, HSO₄-, CHSO₄⁻, H₂PO₄⁻ NO₃⁻

Various commercially available grades of polymer JR are summarized below:

| | **JR-125** | **JR-400** | **JR-30 M** |
|---|---|---|---|
| Brookfield Viscosity at 25°C, centipoises, 2.0 wt.% (1.7 wt.% to 2.2 wt.%) | 110-120 cps | 400-440 cps | 12,000-13,000 cps |

In one embodiment, the cationic polymer is Polymer JR. In another embodiment, the composition comprises a minimum of 0.001 wt.%, about 0.005 wt.%, about 0.01 wt.% or about 0.05 wt.% and/or a maximum of about 0.5 wt.%, about 0.1 wt.% or about 0.05 wt.% Polymer JR (including JR-125, JR-400 and JR-30M) based upon the total weight of the composition. In the case of eye drop solutions, the cationic polysaccharides offer the additional advantage of being further effective as an active agent for treatment of dry eye. In one embodiment, the ratio of zinc compound to polycationic material in the composition is a minimum of about 0.001, about 0.01 or about 0.1 and/or a maximum of about 10, about 5, about 1 or about 0.01.

As mentioned, other polycationic materials besides the cationic polysaccharides may be used in this invention. Examples include: polyquaternium-28, a polyquaternary ammonium salt based on N-vinylpyrrolidone and dimethylaminopropyl methacrylamide monomers (available under the tradename Gafquat HS-100, GAF Chemicals, Wayne, New Jersey, USA); hexadimethrine bromide, a polymer of N,N,N',N'-tetramethylhexamethylene-diamine and trimethylene bromide; hydroxypropyl guar triammonium chloride, a quaternary ammonium derivative of guar gum (available from Carbomer, Inc., Westborough, Massachusetts, USA); copolymers of vinyl caprolactam/PVP/dimethylaminoethyl methacrylate (such as those available under the tradename Gaffix VC-713, GAF Chemicals, Wayne, New Jersey, USA).

Other examples are polymers containing quaternary-amine-functional repeat units, defined as repeat units each comprising a quaternary-amine group, in which a positively charged nitrogen atom is covalently bonded to four radicals (no hydrogen atoms) and ionically bonded to a negatively charged counterion such as chloride.

Specific polyquaternium polymers useful as a polycationic material in the present invention may include, but are not limited to, copolymers in which the quaternary-amine-functional repeat units are derived from one or more of the following kinds of monomers: N,N-dimethyl-N-ethyl-aminoethyl acrylate and methacrylate, 2-methacryloxyethyltrimethylammonium, N-(3-methacrylamidopropyl)-N,N,N-trimethylammonium, 1-vinyl and 3-methyl-1-vinylimidazole, N-(3-acrylamido-3-methylbutyl)-N,N,N-trimethylammonium, N-(3-methacryloyloxy-2-hydroxypropyl)-N,N,N-trimethylammonium, diallyldimethylammonium, diallyldiethylammonium, vinylbenzyltrimethylammonium, their halides or other salt forms, and derivatives thereof.

A specific example of a polyquaternium copolymer is Luviquat^{™} FC 370 polymer (CTFA International Cosmetic Ingredient Dictionary designation polyquaternium-16 commercially available from BASF, Ludwigshafen, Germany) which is the polymerization product of a mixture of comonomers of which 70% is vinylpyrrolidone and 30% is 1-vinylimidazolium methylchloride, commercially available as a composition with a solids content of about 40% by weight in water.

The polycation component may be employed in the compositions in a minimum amount of about 0.001 wt.%, about 0.005 wt.% or about 0.01 wt.% based upon the total weight of the composition and/or a maximum amount of about 1 wt.%, about 0.5 wt.% or about 0.1 wt.% based upon the total weight of the composition.

As noted, the composition has no primary preservative agent. Primary preservative agents include, but are not limited to, sorbic acid. Typically, sorbic acid is added in an amount greater than about 0.15 wt.% based upon the total weight of the composition to be preservative-effective. According to the present invention the sorbic acid is present in a less than preservative-effective amount.

Primary preservative agents include known organic nitrogen-containing agents such as biguanides. The biguanides include the free bases or salts of alexidine, chlorhexidine, hexamethylene biguanides and their polymers, and/or combinations of the foregoing. The biguanide salts are typically gluconates, nitrates, acetates, phosphates, sulfates, halides and the like. A preferred biguanide is the hexamethylene biguanide commercially available from Zeneca, Wilmington, DE under the trademark Cosmocil™ CQ. Generally, the hexamethylene biguanide polymers, also referred to as polyaminopropyl biguanide (PAPB), have molecular weights of up to about 100,000 as disclosed in U.S. Patent No. 5,453,435 and WO 94/04028 the entire contents of which are incorporated herein by reference. Yet another example of a known primary preservative agent is various materials available as polyquaternium-1. Other primary preservative agents include, chlorhexidine as disclosed in U.S. Patent No. 5,453,435; peptides as disclosed in WO 03/006046 and US Publ. 2003/0092612; peroxide and peroxide producing compounds as disclosed in U.S. Patent No. 4,899,914 and WO 02/40062; imidazolium salts as disclosed in 5,200,453; EDTA in combination with scorbic acid as disclosed in U.S. Patent No. 4,529,535; and EDTA in combination with propylene glycol and/or chlorhexidine as disclosed in WO 01/24837, of which all such patents and publications are fully incorporated herein by reference.

The aqueous solutions employed in one embodiment of the present invention contains, in addition to the ingredients described above, one or more other components that are commonly present in ophthalmic solutions, for example, tonicity adjusting agents; buffering agents; chelating agents; pH adjusting agents, viscosity modifying agents, and demulcents and the like, which aid in making ophthalmic compositions more comfortable to the user and/or more effective for their intended use.

The aqueous solutions of the present invention are typically adjusted with tonicity agents to approximate the tonicity of normal lacrimal fluids (approximately equivalent to a 0.9 wt.% solution of sodium chloride or 2.8 wt.% glycerol solution). Typically, the solutions are hypotonic or substantially isotonic with physiological saline used alone or in combination with other adjusting agents. The ophthalmic compositions preferably have an osmolality that is a minimum of about 225 mOsm/kg, about 240 mOsm/kg or about 280 mOsm/kg and/or a maximum of about 400 mOsm/kg, about 360 mOsm/kg, about 340 mOsm/kg or about 320 mOsm/kg.

The compositions may include chelating or sequestering agents in order to chelate or bind metal ions, which might otherwise react with the lens and/or protein deposits and collect on the lens. Examples of such preferred materials may include, but are not limited to, ethylenediaminetetraacetic acid (EDTA) and its salts (disodium), which are usually added in amounts ranging from about 0.01 wt.% to about 0.2 wt.%.

Compositions, such as aqueous solutions, for use in the present invention, may be formulated as lens conditioning solutions or eye-drops and sold in a wide range of small-volume containers from about 1 ml to about 30 ml in size. Such containers can be made from HDPE (high density polyethylene), LDPE (low density polyethylene), polypropylene, poly(ethylene terepthalate) and the like. For eye drops, flexible bottles having conventional dispensing tops are especially suitable for use with the present invention. The eye-drop formulation of the invention is used by instilling, for example, about one (1) or three (3) drops in the eye(s) as needed.

In the case of contact lens treating solutions, the methods and/or compositions of the present invention may be applicable to the conventional contact lens categories: (1) hard lenses formed from materials prepared by polymerization of acrylic esters, such as poly(methyl methacrylate) (PMMA), (2) rigid gas permeable (RGP) lenses formed from silicone acrylates and fluorosilicone methacrylates, (3) soft, hydrogel lenses, (4) silicone hydrogel lenses and (5) non-hydrogel elastomer lenses.

As an example, soft hydrogel contact lenses are made of a hydrogel polymeric material, hydrogels being defined as a cross-linked polymeric systems containing water in an equilibrium state. In general, hydrogels exhibit excellent biocompatibility properties, i.e., the property of being biologically or biochemically compatible by not producing a toxic, injurious or immunological response in a living tissue. Representative conventional hydrogel contact lens materials are made by polymerizing a monomer mixture comprising at least one hydrophilic monomer, such as (meth)acrylic acid, 2-hydroxyethyl methacrylate (HEMA), glyceryl methacrylate, N,N-dimethacrylamide, and N-vinylpyrrolidone (NVP). In the case of silicone hydrogels, the monomer mixture from which the copolymer is prepared further includes a silicone-containing monomer, in addition to the hydrophilic monomer. Generally, the monomer mixture will include a crosslinking monomer, i.e., a monomer having at least two polymerizable radicals, such as ethylene glycol dimethacrylate, tetraethylene glycol dimethacrylate, and methacryloxyethyl vinylcarbonate. Alternately, either the silicone-containing monomer or the hydrophilic monomer may function as a crosslinking agent.

The pH of the composition of one embodiment of the present invention is a minimum of about 5, about 6, about 6.5 or about 7. The pH of composition of one embodiment of the present invention is a maximum of about 7.6 or about 7.8. Suitable buffers may be added, such as borate, citrate, bicarbonate, tris(hydroxymethyl)aminomethane (TRIS-Base), amino alcohols and various mixed phosphate buffers (which may include combinations of Na₂HPO₄, NaH₂PO₄ and KH₂PO₄) and mixtures thereof. Borate buffers are preferred when the primary preservative agent is PHMB. Generally, buffers will be used in amounts having a minimum of about 0.05 wt.% or 0.1 wt.% and/or a maximum of about 1.5 wt.%, 2.0 wt.% or 2.5 wt.% based upon the total weight of the composition.

The therapeutic agents are any agent that, when administered in a therapeutically effective amount, results in a desired local or systemic physiological or pharmacological effect. Ophthalmic therapeutic agents include all ophthalmic agents, which can be topically applied. Such ophthalmic therapeutic agents include, but are not limited to, glaucoma agents, such as beta-blockers, muscarinics and carbonic anhydrase inhibitors; dopaminergic agonists and antagonists; anti-infectives; non-steroidal and steroidal anti-inflammatories, prostaglandins; proteins; growth factors and anti-allergics. Therapeutic agents according to one or more embodiments of the present invention include combinations of two or more ophthalmic agents.

As used herein, the term "ophthalmic composition" denotes a composition intended for application in the eye or intended for treating a medical device to be placed in contact with the eye such as a contact lens. Ophthalmic compositions specifically include compositions for direct instillation in the eye, including eye drop solutions such as for treating dry eye, and contact lens treating solutions distilled directly in the eye such as for rewetting a contact lens while worn. Ophthalmic compositions also include compositions instilled indirectly in the eye, such as contact lens treating solutions for treating the contact lens prior to the lens being inserted on the eye.

The materials suitable for use in the present invention may also be useful as a component of a cleaning, disinfecting or conditioning composition. Such compositions also may include, preservative agents, surfactants, toxicity adjusting agents, buffers and the like that are known to be used components of conditioning and/or cleaning solutions for contact lenses. Examples of suitable formulations for cleaning and/or disinfecting solutions are taught in U.S. Patent 5,858,937 to Richard et al., which is incorporated by reference as if set forth at length herein. Preferably, compositions of the present invention may be formulated as a "multi-purpose solution," meaning that such compositions are used, in one embodiment, for cleaning, chemical disinfection, storing, and rinsing a contact lens. A multi-purpose solution preferably has a viscosity that is a minimum of about 1 cps and/or a maximum of about 25 cps, 50 cps or 75 cps. The total amount of water in the composition is a minimum of about 95 wt.%, 97 wt.% or 98 wt.% based upon the total weight of the composition.

Surfactants, which are suitable for use in the present invention, are classified into cationic surfactants, anionic surfactants, nonionic surfactants and ampholytic surfactants depending upon their dissociation state in their aqueous solutions. Among them, various surfactants, which are classified into cationic surfactants, particularly surfactants which consist of an amino acid derivative, i.e. amino acid type cationic surfactants, have conventionally been proposed as disinfectant cleaning agents or compositions for disinfection. Glycerin may also be included as a component of the present invention. Amphoteric surfactants suitable for use in a composition according to the present invention include materials of the type are offered commercially under the trade name "Miranol." Another useful class of amphoteric surfactants is exemplified by cocoamidopropyl betaine, commercially available from various sources.

Various other surfactants suitable for use in the composition can be readily ascertained, in view of the foregoing description, from McCutcheon's Detergents and Emulsifiers, North American Edition, McCutcheon Division, MC Publishing Co., Glen Rock, N.J. 07452 and the CTFA International Cosmetic Ingredient Handbook, Published by The Cosmetic, Toiletry, and Fragrance Association, Washington, D.C.

Optionally, one or more additional polymeric or non-polymeric demulcents may be combined with the above-named ingredients. Demulcents are known to provide wetting, moisturizing and/or lubricating effects, resulting in increased comfort. Polymeric demulcents can also act as a water-soluble viscosity builder. Included among the water-soluble viscosity builders are the non-ionic cellulosic polymers like methylcellulose, hydroxyethyl cellulose, hydroxypropyl cellulose and carboxymethyl cellulose, poly(N-vinylpyrrolidone), poly(vinylalcohol) and the like. Such viscosity builders or demulcents may be employed in a total amount ranging from about 0.01 to about 5.0 wt.%. Suitably, the viscosity of the final formulation is from about 10 cps to about 50 cps. Comfort agents such as glycerin or propylene glycol can also be added.

### EXAMPLES

The following general procedure was used for evaluating the preservative efficacy (PE) of various eye drop solutions against *Staphylococcus aureus* (ATCC 6538), *Eschrechia coli* (ATCC 8739), *Pseudomonas aeruginosa* (ATCC 9027), *Candida albicans* (ATCC 10231) and *Aspergillus niger* (ATCC 16404) hereinafter collectively referred to as pathogenic organism. This procedure applies to the FDA premarket notification (510(k)) guidance document and ISO/DIS 14730 standard preservative efficacy testing with a 14 day rechallenge. The evaluations were conducted with 3 separate lots of each test solution for each pathogenic organism. Each lot was tested with a different preparation of each pathogenic organism

Bacterial cells were grown on Tryptic Soy Agar (TSA) slants at a temperature ranging from 30°C to 35°C in an incubator for a time period from 18h to 24h. Fungal cells were grown on Sabouraud Dextrose Agar (SDA) slants at a temperature ranging from 20°C to 25°C in an incubator for a time period of 2 to 7 days. Cells were harvested in from 5 ml to 10 ml of Saline (USP, 0.9% saline, with or without 0.1 % tween), which was added to each agar slant, followed by gentle agitation with a sterile cotton swab. The cell suspensions were aseptically dispensed into separate sterile polypropylene centrifuge tubes. Cells were harvested by centrifugation at 3000 rpm for 10 min, washed one time and suspended in Saline TS to a concentration of 2 x 10⁸ cells per ml.

The cell suspension (0.1 ml) was diluted with 20 ml of the article (test solution) to reach a final concentration of from 1.0 x 10⁵ to 1.0 x 10⁶ colony-forming units (CFU). Phosphate Buffered Saline (PBS) was used as a control article. The inoculated test and control articles were incubated at a temperature ranging from 20°C to 25°C in static culture. At time zero, 1 ml of the PB (Phosphate Buffer, USP, pH 7.2) from the control article was diluted with 9 ml of PB and serially diluted cells were plated in triplicate on TSA for bacteria and SDA for fungi. The bacterial plates were incubated at a temperature ranging from 30°C to 35°C for a period ranging from 2 days to 4 days. Fungal plates were incubated at a temperature ranging from 20°C to 25°C for a period ranging from 2 to 7 days.

Similarly, at days 7 and 14, a 1 ml volume from test articles was added into 9 ml of Dey-Engley neutralizing broth (DEB) and serially diluted in DEB and plated in triplicate on TSA for bacteria and SDA for fungi. The bacterial plates were incubated at a temperature ranging from 30°C to 35°C for a period ranging from 2 days to 4 days. Fungal plates were incubated at a temperature ranging from 20°C to 25°C for a period ranging from 2 to 7 days. Developing colonies were counted.

Immediately following the day 14 sampling, test articles were re-inoculated to give final concentrations of from 1.0 x 10⁴ to 1.0 x 10⁵ of each pathogenic organism. At time zero, 1 ml from the inoculum control was added to 9 ml of PB and subsequent serial dilutions were plated in triplicate on TSA for bacteria and SDA for fungi. The bacterial plates were incubated at a temperature ranging from 30°C to 35°C for a period ranging from 2 days to 4 days. Fungal plates were incubated at a temperature ranging from 20°C to 25°C for a period ranging from 2 to 7 days.

At days 21 and 28, 1 ml from the test articles was added to 9 ml of Dey-Engley neutralizing broth (DEB) and again, serial dilutions were plated in triplicate on TSA. Plates were incubated at a temperature ranging from 30°C to 35°C for a period ranging from 2 days to 4 days and developing colonies counted.

Based on the acceptance criteria for bacteria or fungus a solution is acceptable if the number of viable bacteria or fungus recovered per ml is reduced by at least 3.0 logs at day 14 and after the rechallenge at day 14, the concentration of bacteria or fungus is reduced by at least 3.0 logs by day 28.

The results at 14 and 28 days for the tested solutions are shown in the following tables, where "ND" denotes "no detectable bacterial levels."

### Example 1. Base Formulation on PE

**Table 1: Composition and Properties of Example 1**

| Ingredient | WT.% |
|---|---|
| Sodium Chloride | 0.220 |
| Boric Acid | 0.850 |
| Sodium Borate | 0.090 |
| EDTA | 0.050 |
| Polymer JR | 0.02 |
| pH | 7.2-7.4 |
| Osmolality (mOsmo/Kg) | 200-240 |
| PE | Failed |

**Table 2: Detailed Preservative Efficacy Test Result for Example 1**

| | | |
|---|---|---|
| *Staphylococcus aureus* | 7 Days | 1.5 |
| | 14 Days | 2.5 |
| | 21 Days | 0.6 |
| | 28 Days | 1.7 |
| *Pseudomonas aeruginosa* | 7 Days | ND |
| | 14 Days | ND |
| | 21 Days | ND |
| | 28 Days | ND |
| *Eschrechia coli* | 7 Days | 3.6 |
| | 14 Days | 4.5 |
| | 21 Days | 2.7 |
| | 28 Days | 3.6 |
| *Candida albicans* | 7 Days | 0.6 |
| | 14 Days | 2.6 |
| | 21 Days | 0.4 |
| | 28 Days | 1.5 |
| *Aspergillus niger* | 7 Days | 1.5 |
| | 14 Days | 2.5 |
| | 21 Days | 0.0 |
| | 28 Days | -0.3 |

Example 1 shows that the combination of EDTA, a composition with known preservative activity, and Polymer JR did not have sufficient preservative activity to pass the preservative efficacy test in the solution disclosed in Table 1.

### EXAMPLE 2: EFFECT OF ZINC CITRATE ON PE

**Table 3: Composition and Properties of Example 3**

| Ingredient | WT.% |
|---|---|
| Sodium Chloride | 0.220 |
| Boric Acid | 0.850 |
| Sodium Borate | 0.090 |
| EDTA | 0.050 |
| Polymer JR | 0.02 |
| Zinc citrate | 0.05 |
| pH | 7.0-7.4 |
| Osmolality (mOsmo/Kg) | 200-240 |
| PE | Passed |

**Table 4: Detailed Preservative Efficacy Test Result for Example 3**

| | | |
|---|---|---|
| *Staphylococcus aureus* | 7 Days | 4.0 |
| | 14 Days | ND |
| | 21 Days | ND |
| | 28 Days | ND |
| *Pseudomonas aeruginosa* | 7 Days | 1.8 |
| | 14 Days | 4.0 |
| | 21 Days | 2.3 |
| | 28 Days | 3.3 |
| *Eschrechia coli* | 7 Days | 4.8 |
| | 14 Days | ND |
| | 21 Days | 3.8 |
| | 28 Days | ND |
| *Candida albicans* | 7 Days | 0.6 |
| | 14 Days | 1.7 |
| | 21 Days | 0.1 |
| | 28 Days | 0.7 |
| *Aspergillus niger* | 7 Days | ND |
| | 14 Days | ND |
| | 21 Days | 0.2 |
| | 28 Days | 0.1 |

Example 2 discloses the same composition as Example 1, except that 0.05 wt.% of zinc acetate is present. The composition of Example 2 passed the preservative efficacy test. Thus, the addition of zinc acetate improved the preservative efficacy over the composition of Example 1.

### Example 3. Effect of Zinc Chloride on PE

**Table 5: Composition and Properties of Example 3**

| Ingredient | WT.% |
|---|---|
| Sodium Chloride | 0.220 |
| Boric Acid | 0.850 |
| Sodium Borate | 0.090 |
| EDTA | 0.050 |
| Polymer JR | 0.02 |
| Zinc Chloride | 0.05 |
| pH | 7.2-7.6 |
| Osmolality (mOsmo/Kg) | 260-300 |
| PE | Passed |

**Table 6: Detailed Preservative Efficacy Test Results for Example 3**

| | | |
|---|---|---|
| *Staphylococcus aureus* | 7 Days | ND |
| | 14 Days | ND |
| | 21 Days | ND |
| | 28 Days | ND |
| *Pseudomonas aeruginosa* | 7 Days | ND |
| | 14 Days | ND |
| | 21 Days | ND |
| | 28 Days | ND |
| *Eschrechia coli* | 7 Days | ND |
| | 14 Days | ND |
| | 21 Days | ND |
| | 28 Days | ND |
| *Candida albicans* | 7 Days | 0.6 |
| | 14 Days | 2.6 |
| | 21 Days | 0.4 |
| | 28 Days | 1.5 |
| *Aspergillus niger* | 7 Days | 2.2 |
| | 14 Days | 2.7 |
| | 21 Days | 0.4 |
| | 28 Days | 0.2 |

Example 3 discloses the same composition as Example 1, except that 0.05 wt.% of zinc chloride is present. The exact composition is illustrated in Table 5 below. The composition of Example 3 passed the preservative efficacy test even when the composition of Example 1 did not pass the preservative efficacy test. Thus, the addition of zinc chloride improved the preservative efficacy over the composition of Example 1. The detailed results are disclosed in Table 6 below.

### Example 4. Effect of Zinc Chloride on PE

**Table 7: Composition and Properties of Example 4**

| Ingredient | WT.% |
|---|---|
| Sodium Chloride | 0.450 |
| Boric Acid | 0.850 |
| Sodium Borate | 0.090 |
| EDTA | 0.050 |
| Zinc Chloride | 0.050 |
| Polymer JR | 0.02 |
| pH | 7.2-7.4 |
| Osmolality (mOsmo/Kg) | 300-340 |
| PE | Passed |

**Table 8: Detailed Preservative Efficacy Test Results for Example 4**

| | | |
|---|---|---|
| *Staphylococcus aureus* | 7 Days | |
| | 14 Days | ND |
| | 21 Days | |
| | 28 Days | ND |
| *Pseudomonas aeruginosa* | 7 Days | |
| | 14 Days | ND |
| | 21 Days | |
| | 28 Days | ND |
| *Eschrechia coli* | 7 Days | |
| | 14 Days | ND |
| | 21 Days | |
| | 28 Days | ND |
| *Candida albicans* | 7 Days | |
| | 14 Days | 2.3 |
| | 21 Days | |
| | 28 Days | 1.7 |
| *Aspergillus niger* | 7 Days | |
| | 14 Days | 1.6 |
| | 21 Days | |
| | 28 Days | 0.3 |

Example 4 discloses the same composition as Example 3 except the amount of sodium chloride in the composition of Example 4 is approximately twice as much as the amount of sodium chloride in the composition of Example 3 and the osmolality is higher in the composition of Example 4. The composition of Example 4 passed the preservative efficacy test.

Several solutions were made according to the proportions disclosed in Table 9, Table 10 and Table 11. Example 5 represents a solution that is prepared as a control/comparison sample. It contains sodium chloride solution buffered with sodium borate/boric acid. It failed the PE test.

The solution shown in Example 6 is the same as the solution shown in Example 5, except the solution shown in Example 6 contains 0.05 wt.% EDTA. EDTA has known antimicrobial activity but at that solution did not pass the preservative efficacy test.

The solution in Example 7 is the same as the solution in Example 6 except it contains Polymer JR in addition to EDTA. The combination of Polymer JR and EDTA at the concentrations disclosed did not produce a preservative effect at the particular concentrations in the solution of Example 7.

The solution in Example 8 is the same as the solution in Example 6 plus zinc (zinc chloride). The combination of zinc and EDTA passed the preservative efficacy test.

The solution in Example 9 is the same as the solution in Example 8 and further has the same amount of Polymer JR as in Example 7. It passed the preservative efficacy test.

The solution in Example 10 is the same as the solution in Example 9 except that it had one half of the zinc compound found in Example 9. It passed the preservative efficacy test.

The solution in Example 11 is the same as the solution in Example 10 except that it had one-half of the zinc compound found in the solution of Example 10 and one-fourth of the zinc compound found in the solution of Example 9. The osmolality was 231. It failed the preservative efficacy test.

The solution in Example 12 is the same as the solution in Example 11 except that it had approximately one-half of the zinc compound found in the solution of Example 11, approximately one-fourth of the zinc compound found in the solution of Example 10 and approximately one-eighth of the zinc compound found in the solution of Example 9. The osmolality was 224. It failed the preservative efficacy test.

The solution in Example 13 is the same as the solution in Example 10 with the exception that the solution in Example 13 did not have any Polymer JR. The solution in Example 13 did not pass the preservative-efficacy test. The presence of Polymer JR enhances the preservative efficacy of the zinc compounds. The data suggests that a polycationic material, including a cellulosic polymer will enhance the preservative efficacy of a zinc compound. Examples 14 and 15 disclose a solution that is the same as Example 13 except it has a smaller amount of zinc and likewise failed the preservative efficacy test.

The solutions in Examples 16, 17 and 19 are the same as the solutions in Examples 13, 14 and 15, respectively except that the solutions in Examples 16, 17 and 18 did not contain any EDTA. Surprisingly, the solutions without EDTA in Examples 16, 17 and 18 passed the preservative efficacy test while the solutions with zinc and EDTA did not pass.

The solution in Examples 19 and 20 are the same as the control solution in Example 5 except that the solution in Example 19 contained 0.1 wt.% of Polymer JR and the solution in Example 20 contained 0.05 wt.% of Polymer JR without a zinc compound or EDTA. The solutions in Examples 19 and 20 failed the preservative efficacy test.

All of the examples show that zinc compound has preservative efficacy at certain concentrations. The preservative efficacy is enhanced by the presence of polymer JR. The preservative efficacy is not enhanced by the addition of EDTA. A zinc compound alone or in combination with Polymer JR have a preservative effect and are gentle preservatives relative to known preservatives and/or antimicrobial agents.

**Table 9: Composition and Properties of Examples 5 through 10**

| | Example 5 | Example 6 | Example 7 | Example 8 | Example 9 | Example 10 |
|---|---|---|---|---|---|---|
| Ingredients (wt.%) | | | | | | |
| Sodium Borate | 0.090 | 0.090 | 0.090 | 0.090 | 0.090 | 0.090 |
| Boric Acid | 0.850 | 0.850 | 0.850 | 0.850 | 0.850 | 0.850 |
| NaCl | 0.220 | 0.220 | 0.220 | 0.220 | 0.220 | 0.220 |
| EDTA | | 0.050 | 0.050 | 0.050 | 0.050 | 0.050 |
| | | | | | | |
| Zinc | | | | 0.050 | 0.050 | 0.025 |
| Polymer JR | | | 0.020 | | 0.020 | 0.020 |
| pH | 7.32 | 7.22 | 7.22 | 6.96 | 6.97 | 7.00 |
| Osmolality (mOsmo/Kg) | 218 | 222 | 223 | 233 | 235 | 230 |
| | | | | | | |
| Observation before autoclaving | clear | clear | clear | Clear | clear | clear |
| PE Results | Failed | Failed | Failed | Passed | Passed | Passed |

**Table 10: Compositions and Properties of Examples 11-15**

| | Example 11 | Example 12 | Example 13 | Example 14 | Example 15 |
|---|---|---|---|---|---|
| Ingredients (wt.%) | | | | | |
| Sodium Borate | 0.090 | 0.090 | 0.090 | 0.090 | 0.090 |
| Boric Acid | 0.850 | 0.850 | 0.850 | 0.850 | 0.850 |
| NaCl | 0.220 | 0.220 | 0.220 | 0.220 | 0.220 |
| EDTA | 0.050 | 0.050 | 0.050 | 0.050 | 0.050 |
| | | | | | |
| Zinc | 0.0125 | 0.0065 | 0.025 | 0.0125 | 0.0065 |
| Polymer JR | 0.020 | 0.020 | | | |
| | | | | | |
| | | | | | |
| | | | | | |
| pH | 7.1 | 7.19 | 7.12 | 7.20 | 7.22 |
| Osmolality (mOsmo/Kg) | 231 | 224 | 227 | 223 | 223 |
| | | | | | |
| Observation before autoclaving | clear | Clear | Clear | Clear | Clear |
| PE Results | Failed | Failed | Failed | Failed | Failed |

**Table 11: Compositions and Properties of Examples 16-20**

| | Example 16 | Example 17 | Example 18 | Example 19 | Example 20 |
|---|---|---|---|---|---|
| Ingredients (wt.%) | | | | | |
| Sodium Borate | 0.090 | 0.090 | 0.090 | 0.090 | 0.090 |
| Boric Acid | 0.850 | 0.850 | 0.850 | 0.850 | 0.850 |
| NaCl | 0.220 | 0.220 | 0.220 | 0.220 | 0.220 |
| EDTA | | | | 0.050 | 0.050 |
| | | | | | |
| Zinc | 0.025 | 0.0125 | 0.0065 | | |
| Polymer JR | | | | 0.100 | 0.050 |
| | | | | | |
| | | | | | |
| | | | | | |
| pH | 7.40 | 7.44 | 7.43 | 6.78 | 6.96 |
| Osmolality (mOsmo/Kg) | 221 | 219 | 218 | 227 | 221 |
| | | | | | |
| Observation before autoclaving | clear | clear | clear | Clear | Clear |
| PE Results | Passed | Passed | Passed | Failed | Failed |

## Claims

1. An ophthalmic composition comprising:
water;
a soluble amount of a zinc compound,
a cationic cellulosic polymer,
no primary preservative agent,
a buffering agent, and
a chelating agent,
wherein the composition has a minimum of 0.001 wt.-% and a maximum of 1 wt.-% of the zinc compound per total weight of the composition.

2. The composition of claim 1, wherein the composition has a minimum of 0.001 wt.-% and a maximum of 0.5 wt.-% of cationic cellulosic polymer.

3. The composition of claim 2, wherein the cationic cellulosic polymer is Polymer JR.

4. The composition of claim 1, having the form of an eye drop solution.

5. The composition of claim 1, having the form of a contact lens treating solution.

6. The composition of claim 1, being suitable for direct instillation in the eye without causing irritation to eye tissue.

7. The composition of claim 1, further comprising a therapeutic agent is selected from the group consisting of glaucoma agents, muscarinics, carbonic anhydrase inhibitors, dopaminergic agonists and antagonists, anti-infectives, non-steroidal and steroidal anti-inflammatories, prostaglandins, enzymes, growth factors, anti-allergics, beta-blockers and mixtures and combinations thereof.

8. A composition according to any of claims 1 to 7 for use in treating an ophthalmic condition.

## Patentansprüche

1. Eine ophthalmische Zusammensetzung umfassend:
Wasser,
eine lösliche Menge einer Zinkverbindung,
ein kationisches, zellulosehaltiges Polymer,
kein primäres Konservierungsmittel,
eine Puffersubstanz und
einen Chelatbildner,
wobei die Zusammensetzung minimal 0,001 Gew.-% und maximal 1 Gew.-% der Zinkverbindung, bezogen auf das Gesamtgewicht der Zusammensetzung, beinhaltet.

2. Die Zusammensetzung gemäß Anspruch 1, wobei die Zusammensetzung minimal 0,001 Gew.-% und maximal 0,5 Gew.-% des kationischen, zellulosehaltigen Polymers beinhaltet.

3. Die Zusammensetzung gemäß Anspruch 2, wobei das kationische, zellulosehaltige Polymer Polymer JR ist.

4. Die Zusammensetzung gemäß Anspruch 1, in Form einer Augentropfenlösung.

5. Die Zusammensetzung gemäß Anspruch 1, in Form einer Kontaktlinsen-Behandlungs-Lösung.

6. Die Zusammensetzung gemäß Anspruch 1, die für eine direkte Instillation in das Auge geeignet ist, ohne dabei Irritationen des Augengewebes hervorzurufen.

7. Die Zusammensetzung gemäß Anspruch 1, weiterhin umfassend einen Wirkstoff ausgewählt aus der Gruppe bestehend aus Glaukom-Wirkstoffen, Muskarinika, carbonischen Anhydrasehemmern, dopaminergen Agonisten und Antagonisten, Anti-Infektiva, nicht-steroidalen und steroidalen Entzündungshemmern, Prostaglandinen, Enzymen, Wachstumsfaktoren, Anti-Allergenen, Beta-Blockern und Mischungen und Kombinationen daraus.

8. Eine Zusammensetzung gemäß einem der Ansprüche 1 bis 7 zur Verwendung bei der Behandlung einer ophthalmischen Erkrankung.

## Revendications

1. Composition ophtalmique comprenant :
de l'eau ;
une quantité soluble d'un composé de zinc,
un polymère cellulosique cationique,
aucun agent conservateur primaire,
un agent tampon, et
un agent chélatant,
où la composition possède un minimum de 0,001 % pt et un maximum de 1 % pt du composé de zinc par rapport au poids total de la composition.

2. Composition selon la revendication 1, où la composition possède un minimum de 0,001 % pt et un maximum de 0,5 % pt de polymère cellulosique cationique.

3. Composition selon la revendication 2, dans laquelle le polymère cellulosique cationique est un polymère JR.

4. Composition selon la revendication 1, ayant la forme d'une solution de gouttes oculaires.

5. Composition selon la revendication 1, ayant la forme d'une solution de traitement de lentilles de contact.

6. Composition selon la revendication 1, qui est adaptée pour une instillation directe dans l'oeil sans provoquer d'irritation au tissu oculaire.

7. Composition selon la revendication 1, comprenant en outre un agent thérapeutique qui est sélectionné dans le groupe consistant en des agents anti-glaucome, des agents muscariniques, des inhibiteurs de l'anhydrase carbonique, des agonistes et des antagonistes dopaminergiques, des agents anti-infectieux, des agents anti-inflammatoires non stéroïdiens et stéroïdiens, des prostaglandines, des enzymes, des facteurs de croissance, des agents anti-allergiques, des bêta-bloquants et des mélanges et des combinaisons de ceux-ci.

8. Composition selon l'une quelconque des revendications 1 à 7, destinée à être utilisée dans le traitement d'une affection ophtalmique.
